# FASCICULE DE BREVET EUROPEEN

(11) **EP 1 010 694 B1**
(45) Date de publication et mention de la délivrance du brevet: **06.08.2003**
(21) Numéro de dépôt: 99403189.6
(22) Date de dépôt: 17.12.1999
(51) Int. Cl.: C07D 235/02, A61K 31/4184, A61P 25/24

(54) **Dérivés spiroimidazoliniques et leur utilisation comme antagonistes alpha2-adrénergiques et bloqueurs de la recapture de monoamines**
Spiroimidazolinderivate und ihre Verwendung als alpha2-Adrenorezeptor-Antagonisten und Monoamino-Wiederaufnahme-Blocker
Spiroimidazoline derivatives and their use as alpha2-adrenergic antagonists and monoamines-reuptake blockers

(30) Priorité: 18.12.1998 FR 9816000
(43) Date de publication de la demande: 21.06.2000
(73) Titulaire: LES LABORATOIRES SERVIER, 92200 Neuilly sur Seine (FR)
(72) Inventeur: Cordi, Alex, 92150 Suresnes (FR); Millan, Mark, 78230 Le Pecq (FR); Newman-Tancredi, Adrian, 78230 Le Pecq (FR); Brocco, Mauricette, 75003 Paris (FR)

(56) Documents cités:
- EP-A- 0 635 497
- CHEMICAL ABSTRACTS, vol. 113, no. 15, 8 octobre 1990 (1990-10-08) Columbus, Ohio, US; abstract no. 126536z, PERRONE M H ET AL: "In vivo assessment of napamezole, an alpha-2 adrenoreceptor antagonist and monoamine re-uptake inhibitor" page 72; XP002136506 & J. PHARMACOL. EXP. THER., vol. 254, no. 2, 1990, pages 476-83,

## Description

La présente invention concerne de nouveaux dérivés spiroimidazoliniques et les compositions pharmaceutiques qui les contiennent, ainsi que leur utilisation en tant qu'antagonistes α2-adrénergiques et bloqueurs de la recapture de monoamines.

Le système nerveux adrénergique joue un rôle important à plusieurs niveaux, par exemple artériel, veineux, cardiaque, rénal et au niveau du système nerveux autonome central et périphérique. Dès lors, les produits capables d'interagir avec les récepteurs adrénergiques peuvent induire un grand nombre de réponses physiologiques comme la vasoconstriction, la vasodilatation, l'augmentation ou la diminution du rythme cardiaque, la variation de la force de contraction du muscle cardiaque et des activités métaboliques. Différents composés adrénergiques ont été utilisés dans le passé pour modifier ces réponses physiologiques ou d'autres.

On trouve dans l'art antérieur des composés spiroimidazoliniques utiles en tant qu'agonistes ou agonistes partiels α1 ou α2-adrénergiques (EP 635495, EP 635496, EP 635497).

Les composés décrits dans la présente invention, outre le fait qu'ils sont nouveaux, possèdent un profil d'antagonistes α2-adrénergiques et de bloqueur de la recapture de monoamines, les rendant utiles dans le traitement de la dépression (Drug News & Perspectives, 4 (4), 1991). Le problème majeur posé par les antidépresseurs est leur long délai d'efficacité, lié à leur propre mode d'action. Des études ont montré que l'association d'un antagoniste α2-adrénergique avec un inhibiteur de la recapture de monoamines (serotonine et/ou noradrénaline) permettait de réduire ce délai (Commun. Psychopharmacol, 4, pp. 95-100, 1980). La combinaison des deux effets sur une seule molécule pourrait donner naissance à une nouvelle génération d'antidépresseurs beaucoup plus efficaces. Parmi ces composés, le napamezole (US 5017584) est décrit comme possédant à la fois une activité antagoniste α2-adrénergique et bloqueur de la recapture de monoamines.

Les composés de la présente invention, de structure nouvelle, présentent à la fois un profil sélectif d'antagonistes α2- adrénergiques et un pouvoir d'inhibiteurs de la recapture de monoamines.

La présente invention concerne plus particulièrement les composés de formule (I): 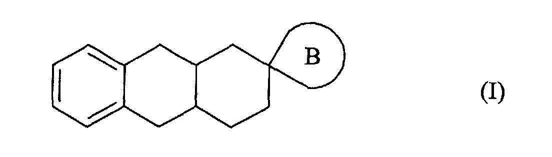 dans laquelle :
◆ B représente un cycle imidazolinique tel que représenté dans les formules (Ia) ou (Ib) : 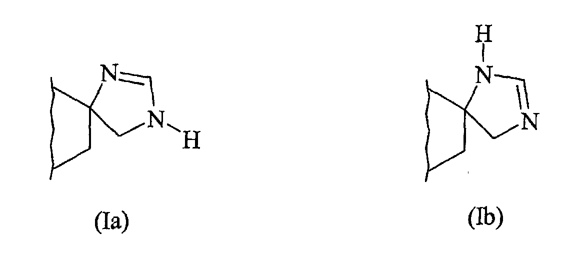
leurs tautomères, énantiomères et diastéréoisomères, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, l'invention concerne les composés de formule (I) dans lesquels B représente un cycle de formule (la).

Très avantageusement, l'invention concerne les composés de formule (I) à jonction de cycle trans.

Encore plus particulièrement, l'invention concerne le spiro[(1,3-diazacyclopent-1-ène)-5:2'-(*trans*-1',2',3',4',4'a,9',9'a,10'-octahydroanthracène)], et préférentiellement, le mélange constitué du spiro[(1,3-diazacyclopent-1-ène)-5:2'*(S)*-(*trans*-1',2',3',4',4'a*(R)*, 9',9'a*(S)*,10'-octahydroanthracène)] et de son énantiomère, et le mélange constitué du spiro[(1,3-diazacyclopent-1-ène)-5:2'*(S)*-(*trans*-1',2',3',4',4'a*(S)*,9',9'a*(R)*, 10'-octahydroanthracène)] et de son énantiomère.

Les tautomères, énantiomères et diastéréoisomères ainsi que les sels d'addition à un acide ou à une base pharmaceutiquement acceptable des composés préférés de l'invention font partie intégrante de l'invention.

L'invention concerne également le procédé de préparation des composés de formule (I) caractérisé en ce que l'on utilise comme produit de départ le composé de formule (II) : 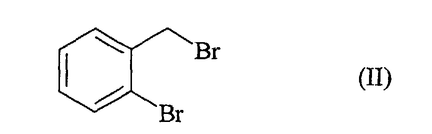 sur lequel on condense l'énolate de la 1,4-cyclohexanedione-mono éthylène acétal afin d'obtenir le composé de formule (III) : 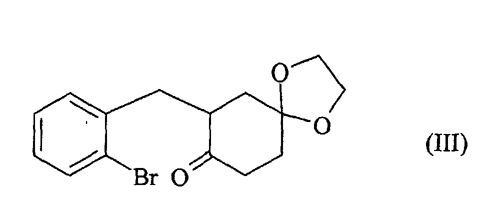 qui est soumis à l'action d'iodure de méthyl(triphényl)phosphonium en milieu basique pour conduire au composé de formule (IV) : 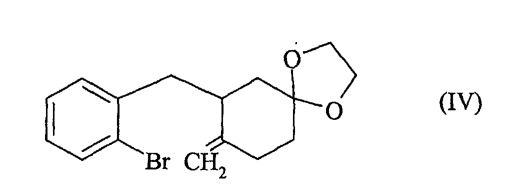 qui est cyclisé en présence d'hydrure de tributylétain et d'AIBN pour conduire au composé de formule (V) : 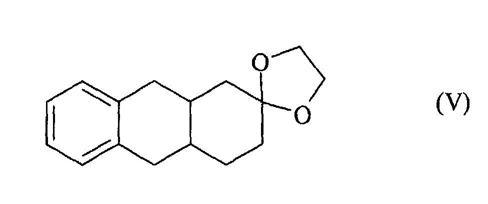 que l'on soumet successivement à l'action d'un milieu acide puis à la réaction de Strecker pour obtenir le composé de formule (VI) : 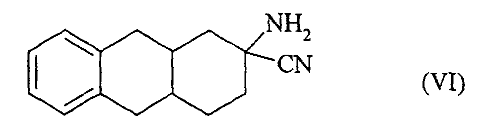 que l'on soumet à l'action d'un agent réducteur comme LiAlH₄ par exemple pour conduire au composé de formule (VII) : 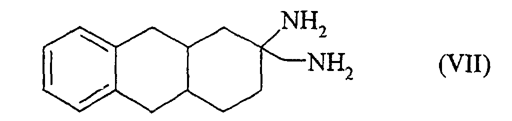 qui est mis en réaction avec de l'acétate de formamidine pour obtenir le composé de formule (I/a), cas particulier des composés de formule (I) : 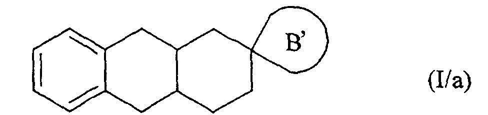 dans laquelle B' représente un cycle imidazolinique non substitué tel que représenté dans les formules (Ia/a) et (Ib/a) : 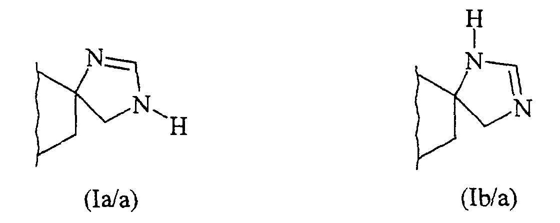 que l'on peut soumettre, en présence d'une base, à l'action d'un composé de formule (VIII):

R'-J (VIII)

dans laquelle R' représente un groupement alkyle (C₁-C₆) linéaire ou ramifié ou benzyle et J représente un groupe partant comme un atome d'halogène ou un groupement tosyle, pour conduire au composé de formule (I/b), cas particulier des composés de formule (I) : 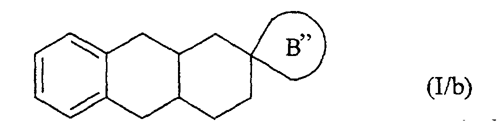 dans laquelle B" représente un cycle imidazolinique substitué tel que représenté dans les formules (Ia/b) et (Ib/b) : 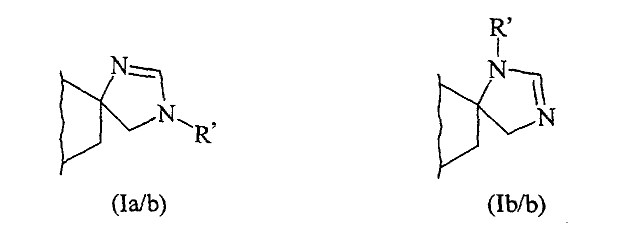 où R' est tel que défini précédemment,
les composés de formule (I/a) et (I/b) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

Les composés de l'invention et les compositions pharmaceutiques les contenant s'avèrent être utiles pour le traitement de la dépression.

En effet, les composés de la présente invention sont des antagonistes spécifiques α2-adrénergiques et agissent également comme de puissants inhibiteurs de recapture de la sérotonine et/ou de la noradrénaline.

A ce titre, ils peuvent être utilisés en thérapeutique pour le traitement de la dépression, de l'obésité, des attaques de panique, de l'anxiété, des troubles obsessionnels compulsifs, des troubles cognitifs, des phobies, des troubles impulsifs de l'abus et du sevrage de drogue, des dysfonctionnements sexuels et de la maladie de Parkinson.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques et les ampoules buvables ou injectables.

La posologie varie selon le sexe, l'âge et le poids du patient, la voie d'administration, la nature de l'indication thérapeutique, ou des traitements éventuellement associés et s'échelonne entre 1 et 1000 mg par 24 heures en 1 ou plusieurs prises.

Les exemples suivants illustrent l'invention et ne la limitent en aucune façon.

### EXEMPLE 1 : Spiro[(1,3-diazacyclopent-1-ène)-5:2'(S)-(trans-1',2',3',4',4'a(R),9', 9'a(S),10'-octahydroanthracène)],fumarate et Spiro[(1,3-diazacyclopent-1-ène)-5:2'(R)-(trans-1',2',3',4',4'a(S),9', 9'a(R),10'-octahydroanthracène)],fumarate

### Stade 1 : 7-(2-Bromobenzyl)-1,4-dioxaspiro[4.5]decan-8-one

A une solution de diisopropylamidure de Lithium 1M dans du THF (150 mmol, 150 ml) refroidie à -78°C sous azote est ajoutée goutte à goutte une solution de 1,4-cyclohexanedione mono-éthylène acétal (20 g, 28 mmol) dans du THF (360 ml), puis on laisse revenir à température ambiante. Après 1 heure d'agitation, le milieu est refroidi à -78°C et 35,2 g (141 mmol) de bromure de 2-bromobenzyl sont additionnés goutte à goutte. Après 30 minutes d'agitation à -78°C, la température du milieu est remontée à 0°C. Le mélange est agité 3 heures à 0°C puis hydrolysé et extrait à l'éther. La phase organique est lavée avec une solution saturée de NaCl, séchée sur MgSO₄ et concentrée sous vide. Le composé du titre est purifié par chromatographie sur colonne flash.
Point de fusion: 123°C

### Stade 2 : 7-(2-Bromobenzyl)-8-méthylène-1,4-dioxaspiro[4.5]décane

A une suspension d'iodure de méthyl(triphényl)phosphonium (25 g, 61,8 mmol) dans 50 ml de toluène est additionnée une solution de *t*-pentoxide de sodium (70 ml d'une solution 1M) préparée extemporanément, et le milieu est agité à température ambiante sous azote pendant 20 minutes. Le composé obtenu au stade 1 (6,70 g, 20,6 mmol) en solution dans 50 ml de toluène est ajouté goutte à goutte et la réaction portée au reflux pendant 3 heures. Après refroidissement, la réaction est hydrolysée avec une solution saturée de NH₄Cl et extraite à l'éther. La phase organique est lavée avec une solution saturée de NaCl, H₂O, séchée sur MgSO₄ et concentrée sous vide. Le produit du titre est purifié par chromatographie sur colonne flash (SiO₂, toluène / cyclohexane : 60 / 40).
Point de fusion : 68°C

### Stade 3 : 2-Dioxolan-trans-1,2,3,4,4a,9,9a,10-octahydroanthracène

Une solution contenant le composé obtenu au stade 2 (5 g, 15,5 mmol), 510 mg (0,02 mmol) d'AIBN et 6,75 g de Bu₃SnH (23,2 mmol) dans 750 ml de toluène est portée à reflux *5* heures 30 sous azote. Le solvant est évaporé sous pression réduite et le résidu obtenu est agité vigoureusement pendant 3 heures avec un mélange d'éther (120 ml) et une solution saturée de fluorure de potassium (120 ml). Après filtration, extraction à l'éther, séchage sur MgSO₄ et concentration sous pression réduite, le produit du titre est purifié par chromatographie sur colonne flash (SiO₂, cyclohexane / éther : 80 / 20).
Point de fusion : 71°C

### Stade 4 : Trans-1,2,3,4,4a,9,9a,10-octahydro-2-anthracènone

Une solution de 6 g (24,6 mmol) de l'acétal obtenu au stade 3 dans 100 ml d'acétone et 25 ml d'eau, et 1,85 g (7,4 mmol) de tosylate de pyridinium est portée au reflux pendant 4 heures. L'excès de solvant est évaporé sous vide puis 500 ml d'éther sont ajoutés et le milieu réactionnel est lavé avec une solution saturée de Na₂CO₃ et une solution saturée de NaCl. La phase organique est séchée sur MgSO₄ et le solvant est évaporé sous pression réduite. Le composé du titre est obtenu après purification par chromatographie sur colonne flash (SiO₂, cyclohexane / acétate d'éthyle : 80 / 20).
Point de fusion : 99°C

### Stade 5 : 2-Amino-trans-1,2,3,4,4a,9,9a,10-octahydro-2-anthracène carbonitrile

A une solution contenant 1,25 g (6,2 mmol) du composé obtenu au stade 4 dans 30 ml de MeOH et 15 ml d'eau agitée vigoureusement et maintenue sous azote, sont ajoutés successivement 410 mg de KCN (6,3 mmol) et 340 mg de NH₄Cl (6,3 mmol). Après 12 heures d'agitation à 20°C, la solution est diluée dans du CH₂Cl₂ et extraite au CH₂Cl₂. La phase organique est lavée avec une solution saturée de NaCl, séchée sur MgSO₄ et évaporée. Le résidu est traité avec une solution méthanolique d'ammoniac 7N (25 ml) et agitée en système clos 12 heures à 20°C. Après évaporation sous pression réduite, le composé du titre est obtenu pur.
Point de fusion : 128°C

| Microanalyse élémentaire : | | | |
|---|---|---|---|
| | C% | H% | N% |
| Théorique | 79,61 | 8,02 | 12,38 |
| Trouvée | 79,88 | 8,18 | 12,60 |

### Stade 6 : 2-Aminométhyl-trans-1,2,3,4,4a,9,9a,10-octahydro-2-anthracènamine

Une solution de 1,39 g (6,1 mmol) du nitrile obtenu au stade 5 dans 35 ml de THF est additionnée goutte à goutte à une suspension de LiAlH₄ (350 mg, 9,2 mmol) dans 35 ml de THF anhydre à -20°C sous azote. Le milieu est agité 1 heure 30 avant d'être hydrolysé par 2,3 ml d'H₂O, 4,6 ml de soude à 35% et 4,9 ml d'eau. La suspension obtenue est filtrée et le filtrat évaporé pour conduire à une huile qui est chromatographiée sur colonne flash : le composé du titre est isolé sous forme de mélange de deux diastéréoisomères, séparables sur HPLC (Kromasil 100,10 C18-210 mm-CH₃CN / H₂O / CF₃COOH = 170 / 830 / 5).

(*(2S)*-2-aminométhyl-*trans*-1,2,3,4,4a*(R)*,9,9a*(S)*,10-octahydro-2-anthracènamine et *(2R)*-2-aminométhyl-*trans*-1,2,3,4,4a*(S*),9,9a*(R)*,10-octahydro-2-anthracénamine)
Point de fusion : 123°C

(*(2S)*-2-aminométhyl-*trans*-1,2,3,4,4a*(S)*,9,9a*(R)*,10-octahydro-2-anthracènamine et *(2R)*-2-aminométhyl-*trans*-1,2,3,4,4a*(R)*,9,9a*(S)*, 10-octahydro-2-anthracènamine)
Point de fusion : 183°C

### Stade 7 : Spiro[(1,3-diazacyclopent-1-ène)-5:2'(S)-(trans-1',2',3',4',4'a(R),9', 9'a(S),10'-octahydroanthracène)],fumarate et Spiro[(1,3-diazacyclopent-1-ène)-5:2'(R)-(trans-1',2',3',4',4'a(S),9', 9'a(R),10'-octahydroanthracène)],fumarate

Un mélange de 495 mg (2,2 mmol) de *(2S)*-2-aminométhyl-*trans*-1,2,3,4,4a*(R)*,9,9a*(S)*, 10-octahydro-2-anthracènamine et *(2R)*-2-aminométhyl-*trans*-1,2,3,4,4a*(S)*,9,9a*(R)*, 10-octahydro-2-anthracènamine obtenues au stade 6, et 258 mg (2,5 mmol) d'acétate de formamidine dans 10 ml d'EtOH est agité à 20°C sous azote pendant 12 heures. Le solvant est évaporé et le résidu repris dans HCl 1N. La phase acide est lavée à l'éther, basifiée avec NaOH 35% puis extraite avec CH₂Cl₂. La phase organique est lavée avec une solution saturée de NaCl, séchée sur MgSO₄ et évaporée. Le résidu solide est dissout dans 10 ml d'EtOH et traité par une solution d'acide fumarique (225 mg, 1,9 mmol) dans 10 ml d'EtOH. Après évaporation et recristallisation dans EtOH, le composé du titre est obtenu sous forme d'une poudre blanche.
Point de fusion : 233-237°C

### EXEMPLE 2 : Spiro[(1,3-diazacyclopent-1-ène)-5:2'(S)-(trans-1',2',3',4',4'a(S),9', 9'a(R),10'-octahydroanthracène)],fumarate et Spiro[(1,3-diazacyclopent-1-ène)-5:2'(R)-(trans-1',2',3',4',4'a(R),9', 9'a(S),10'-octahydroanthracène)],fumarate

On procède comme dans le stade 7 de l'Exemple 1 à partir de la *(2S)*-2-aminométhyl-*trans*-1,2,3,4,4a*(S)*,9,9a*(R)*,10-octahydro-2-anthracénamine et de son énantiomère.
Point de fusion: 215°C

### ETUDE PHARMACOLOGIQUE

### EXEMPLE A : Détermination de l'affinité pour les récepteurs adrénergiques α₂ chez le rat

L'affinité a été déterminée par des expériences de compétition avec le [³H]-RX 821,002. Les membranes sont préparées à partir de cortex cérébral de rat et incubées en triple avec 0,4 nM de [³H]-RX 821,002 et le produit à tester dans un volume final de 1,0 ml, pendant 60 minutes à 22°C. Le tampon d'incubation contient 50 nM de TRIS-HCl (pH 7,5), 1 mM de EDTA et 100 µM de GppNHp. La fixation non spécifique est déterminée avec 10 µM de phentolamine.

### Analyse de données

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

### Résultat

Les composés de l'invention montrent une activité antagoniste spécifique des récepteurs α2-adrénergiques avec, par exemple pour le composé de l'Exemple 1 un pKi de 8,0.

### EXEMPLE B: Détermination de l'affinité pour les sites de recapture de la noradrenaline chez le rat

L'affinité a été déterminée par des expériences de compétition avec [³H]-nisoxetine. Les membranes sont préparées à partir de cortex frontal de rat et incubées en triple avec 2 nM de [³H]-nisoxetine et le produit à tester dans un volume final de 0,5 ml, pendant 4 heures à 4°C. Le tampon d'incubation contient 50 mM de TRIS-HCl (pH 7,4), 120 mM de NaCl et 5 mM de KCl. La fixation non-spécifique est déterminée avec 10 µM de desipramine.

### Analyse de données

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

### Résultat

Les composés de la présente invention montrent une très bonne affinité pour les sites de recapture de la noradrénaline. A titre d'Exemple, le pKi du composé de l'Exemple 1 est de 6,7.

### EXEMPLE C : Détermination de l'affinité pour les sites de recapture de la sérotonine chez le rat

L'affinité a été déterminée par des expériences de compétition avec le [³H]-paroxetine. Les membranes sont préparées à partir de cortex frontal de rat et incubées en triple avec 0,25 nM de [³H]-paroxetine et le ligand froid dans un volume final de 0,4 ml, pendant 2 heures à 25°C. Le tampon d'incubation contient 50 mM de TRIS-HCl (pH 7,4), 120 mM de NaCl et 5 mM de KCI. La fixation non-spécifique est déterminée avec 10 µM de citalopram.

### Analyse de données

A la fin de l'incubation, le milieu d'incubation est filtré au travers de filtres WHATMAN GF/B imprégnés avec 0,1 % de polyéthylénimine et lavé trois fois avec 5 ml de tampon refroidi. La radioactivité retenue sur les filtres est déterminée par comptage du liquide de scintillation. Les isothermes de binding sont analysées par régression non-linéaire.

### Résultat

Les composés de la présente invention montrent une très bonne affinité pour les sites de recapture de la sérotonine. A titre d'Exemple, le pKi du composé de l'Exemple 1 est de 7,8.

### EXEMPLE D : Composition pharmaceutique : Comprimés

| 1000 comprimés dosés à 5 mg | |
|---|---|
| Composé de l'Exemple 1 | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 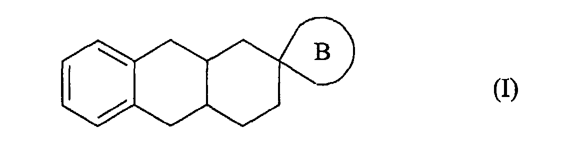 dans laquelle :
◆ B représente un cycle imidazolinique tel que représenté dans les formules (Ia) ou (Ib) : 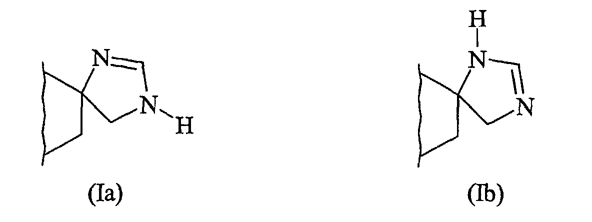
leurs tautomères, énantiomères et diastéréoisomères, ainsi que leurs sels d'addition à un acide ou à une base pharmaceutiquement acceptable.

2. Composés de formule (I) selon la revendication 1 présentant une jonction de cycle trans, leurs tautomères, énantiomères et diastéréoisomères, ainsi que leurs sels d'addition à un acide ou à une base pharmaceutiquement acceptable.

3. Composé de formule (I) selon la revendication 1 qui est le spiro[(1,3-diazacyclopent-1-ène)-5:2'-(*trans*-1',2',3',4',4'a,9',9'a,10'-octahydroanthracène)], ses tautomères, énantiomères et diastéréoisomères, ainsi que ses sels d'addition à un acide ou à une base pharmaceutiquement acceptable.

4. Composé de formule (I) selon la revendication 1 constitué du mélange du spiro [(1,3-diazacyclopent-1-ène)-5:2'*(S)*-(*trans-*1',2',3',4',4'a*(R)*,9',9'a*(S)*, 10'-octahydroanthracène)] et du spiro[(1,3-diazacyclopent-1-ène)-5:2'*(R)*-(*trans*-1',2',3',4',4'a*(S)*, 9',9'a*(R)*,10'-octahydroanthracène)], ses tautomères, énantiomères et diastéréoisomères, ainsi que ses sels d'addition à un acide ou à une base pharmaceutiquement acceptable

5. Composé de formule (I) selon la revendication 1 constitué du mélange du spiro [(1,3-diazacyclopent-1-ène)-5:2'*(S)*-(*trans*-1',2',3',4',4'a*(S)*,9',9'a*(R)*,10'-octahydroanthracène)] et du spiro[(1,3-diazacyclopent-1-ène)-5:2'*(R)-*(*trans-*1',2',3',4',4'a*(R)*, 9',9'a*(S)*,10'-octahydroanthracène)], ses tautomères, énantiomères et diastéréoisomères, ainsi que ses sels d'addition à un acide ou à une base pharmaceutiquement acceptable.

6. Procédé de préparation des composés de formule (I) selon la revendication 1 **caractérisé en ce que** l'on utilise comme produit de départ un composé de formule (II) : 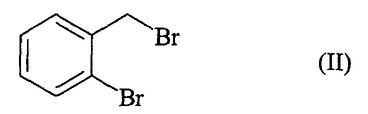 sur lequel on condense l'énolate de la 1,4-cyclohexanedione-mono éthylène acétal afin d'obtenir le composé de formule (III) : 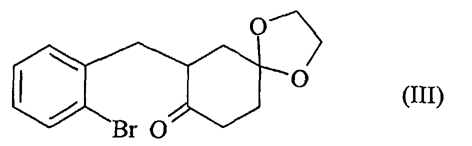 qui est soumis à l'action d'iodure de méthyl(triphényl)phosphonium en milieu basique pour conduire au composé de formule (IV) : 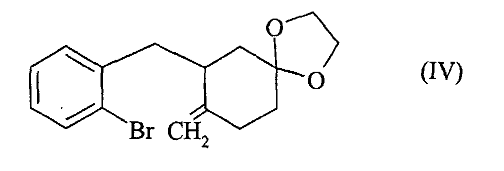 qui est cyclisé en présence d'hydrure de tributylétain et d'AIBN pour conduire au composé de formule (V) : 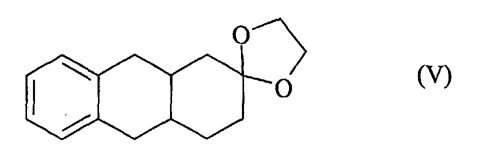 que l'on soumet successivement à l'action d'un milieu acide puis à la réaction de Strecker pour obtenir le composé de formule (VI) : 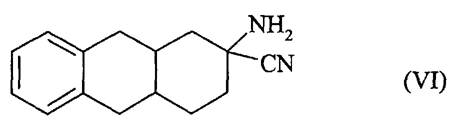 que l'on soumet à l'action d'un agent réducteur pour conduire au composé de formule (VII) : 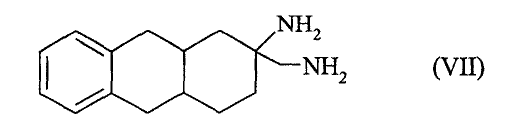 qui est mis en réaction avec de l'acétate de formamidine pour obtenir le composé de formule (I/a), cas particulier des composés de formule (I) : 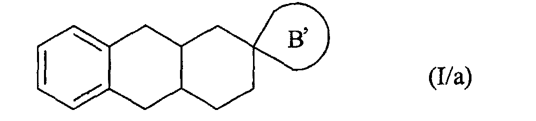 dans laquelle B' représente un cycle imidazolinique non substitué tel que représenté dans les formules (Ia/a) et (Ib/a) : 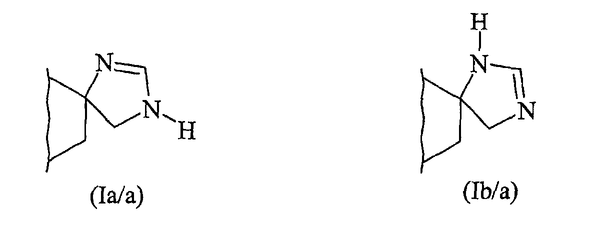 que l'on peut soumettre, en présence d'une base, à l'action d'un composé de formule (VIII) :
R'-J (VIII)
dans laquelle R' représente un groupement alkyle (C₁-C₆) linéaire ou ramifié ou benzyle et J représente un groupe partant comme un atome d'halogène ou un groupement tosyle, pour conduire au composé de formule (I/b), cas particulier des composés de formule (I) : 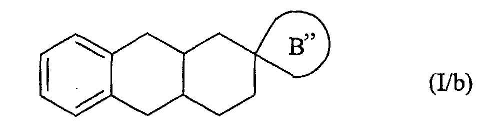 dans laquelle B" représente un cycle imidazolinique substitué tel que représenté dans les formules (Ia/b) et (Ib/b) : 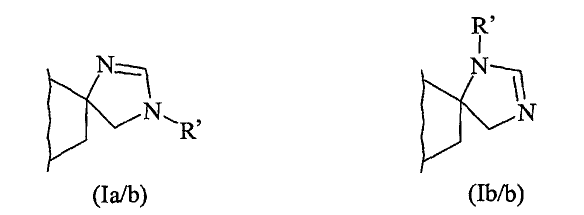 où R' est tel que défini précédemment,
les composés de formule (I/a) et (I/b) formant l'ensemble des composés de formule (I) et pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

7. Compositions pharmaceutiques contenant comme principe actif au moins un des composés de formule (I) selon l'une quelconque des revendications 1 à 5 ou un de ses sels d'addition avec un acide ou une base pharmaceutiquement acceptable en combinaison avec un ou plusieurs excipients pharmaceutiquement acceptables.

8. Compositions pharmaceutiques selon la revendication 7 utiles pour la fabrication d'un médicament pour le traitement de la dépression, de l'obésité, des attaques de panique, de l'anxiété, des troubles obsessionnels compulsifs, des troubles cognitifs, des phobies, des troubles impulsifs de l'abus et du sevrage de drogue, des dysfonctionnements sexuels et de la maladie de Parkinson.

## Patentansprüche

1. Verbindungen der Formel (I): 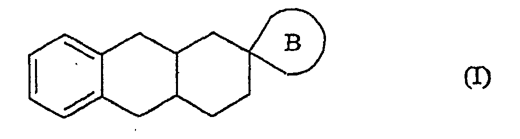 in der
◆ B einen Imidazolinring bedeutet, wie er in den Formeln (Ia) oder (Ib) dargestellt ist: 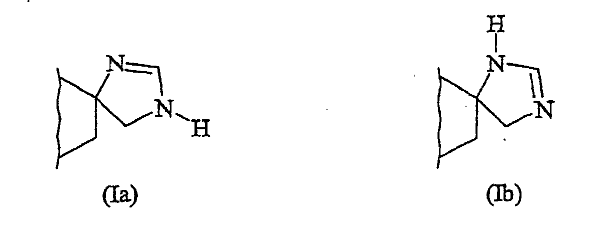
deren Tautomere, Enantiomere und Diastereoisomere sowie deren Additionssalze mit einer pharmazeutisch annehmbaren Säure oder Base.

2. Verbindungen der Formel (I) nach Anspruch 1 mit einer trans-Verbindung des Ringes, deren Tautomere, Enantiomere und Diastereoisomere sowie deren Additionssalze mit einer pharmazeutisch annehmbaren Säure oder Base.

3. Verbindung der Formel (I) nach Anspruch 1, nämlich Spiro[(1,3-diazacyclopent-1-en)-5:2'-(*trans*-1',2',3',4',4'a,9',9'a,10'-octahydroanthracen)], dessen Tautomere, Enantiomere und Diastereoisomere sowie dessen Additionssalze mit einer pharmazeutisch annehmbaren Säure oder Base.

4. Verbindung der Formel (I) nach Anspruch 1 in Form einer Mischung aus Spiro[(1,3-diazacyclopent-1-en)-5:2'(*S*)-(*trans*-1',2',3',4',4'a(*R*),9',9'a(*S*),10'-octahydroanthracen)] und Spiro[(1,3-diazacyclopent-1-en)-5:2'(*R*)-(*trans*-1',2',3',4',4'a(*S*), 9',9'a(*R*),10'-octahydroanthracen)], deren Tautomere, Enantiomere und Diastereoisomere sowie deren Additionssalze mit einer pharmazeutisch annehmbaren Säure oder Base.

5. Verbindung der Formel (I) nach Anspruch 1 in Form einer Mischung aus Spiro[(1,3-diazacyclopent-1-en)-5:2',(*S*)-(*trans*-1',2',3',4',4'a(*S*),9',9'a(*R*),10'-octahydroanthracen)] und Spiro[(1,3-diazacyclopent-1*-*en)-5:2'(*R*)*-*(*trans-*1',2',3',4',4'a(*R*), 9',9'a(*S*),10'-octahydroanthracen)], deren Tautomere, Enantiomere und Diastereoisomere sowie deren Additionssalze mit einer pharmazeutisch annehmbaren Säure oder Base.

6. Verfahren zur Herstellung der Verbindungen der Formel (I) nach Anspruch 1, **dadurch gekennzeichnet, daß** man als Ausgangsprodukt eine Verbindung der Formel (II) verwendet: 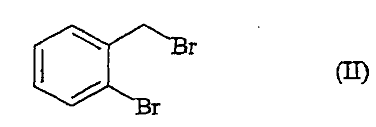 welche man mit dem Enolat von 1,4-Cyclohexandion-mono-ethylenacetal kondensiert zur Bildung der Verbindung der Formel (III): 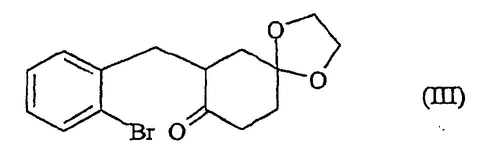 welche man der Einwirkung von Methyl-(triphenyl)-phosphoniumiodid in basischem Medium unterwirft zur Bildung der Verbindung der Formel (IV): 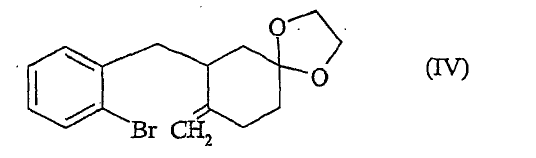 welche man in Gegenwart von Tributylzinn und AIBN cyclisiert zur Bildung der Verbindung der Formel (V): 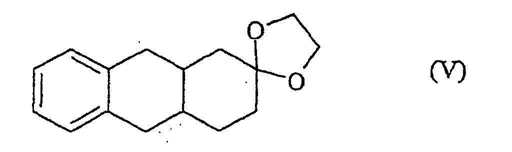 welche man nacheinander der Einwirkung eines sauren Mediums und dann der Strecker-Reaktion unterwirft zur Bildung der Verbindung der Formel (VI): 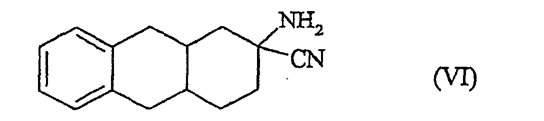 welche man der Einwirkung eines Reduktionsmittels unterzieht zur Bildung der Verbindung der Formel (VII): 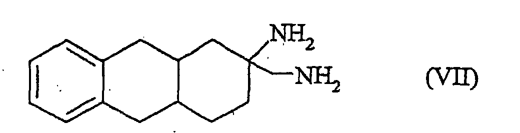 welche man der Einwirkung von Formamidinacetat unterwirft zur Bildung der Verbindung der Formel (I/a), einem Sonderfall der Verbindungen der Formel (I): 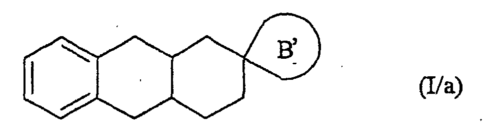 in der B' einen nichtsubstituierten Imidazolinring darstellt, wie er in den Formeln (Ia/a) und (Ib/a) dargestellt ist: 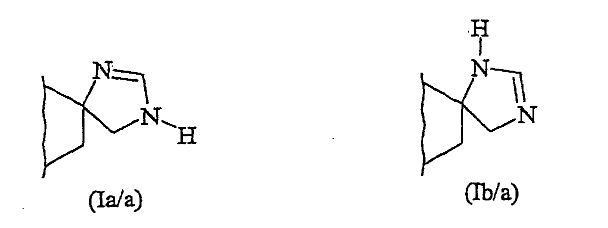 welche man in Gegenwart einer Base der Einwirkung einer Verbindung der Formel (VIII) unterwirft:
R'-J (VIII)
in der R' eine geradkettige oder verzweigte (C₁-C₆)-Alkylgruppe oder Benzylgruppe und J eine austretende Gruppe, wie ein Halogenatom oder eine Tosylgruppe bedeuten, zur Bildung der Verbindung der Formel (I/b), einem Sonderfall der Verbindungen der Formel (I): 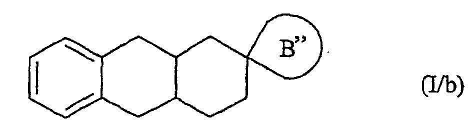 in der B" einen substituierten Imidazolinring bedeutet, wie er in den Formeln (Ia/b) und (Ib/b) dargestellt ist: 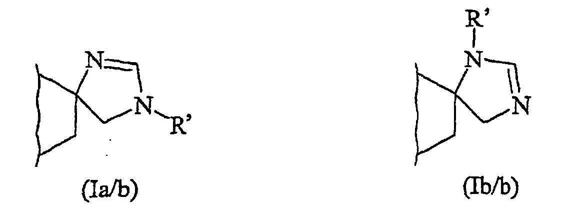 worin R' die oben angegebenen Bedeutungen besitzt,
welche Verbindungen der Formeln (I/a) und (I/b), welche die Gesamtheit der Verbindungen der Formel (I) bilden, mit Hilfe einer klassischen Trennungsmethode gereinigt werden können, gewünschtenfalls in ihre Additionssalze mit einer pharmazeutisch annehmbaren Säure oder Base überführt werden können und gegebenenfalls mit Hilfe einer klassischen Trennungsmethode in ihre Isomeren aufgetrennt werden können.

7. Pharmazeutische Zubereitungen enthaltend als Wirkstoff mindestens eine der Verbindungen der Formel (I) nach einem der Ansprüche 1 bis 5 oder eines ihrer Additionssalze mit einer pharmazeutisch annehmbaren Säure oder Base in Kombination mit einem oder mehreren pharmazeutisch annehmbaren Trägermaterialien.

8. Pharmazeutische Zubereitungen nach Anspruch 7 nützlich für die Herstellung eines Arzneimittels zur Behandlung von Depressionen, der Fettsucht, von Panikanfällen, der Angst, von krampfartigen Besessenheitsstörungen, von Erkenntnisstörungen, Phobien, impulsiven Störungen des Mißbrauchs und des Entzugs von Drogen, sexuellen Dysfunktionen und der Parkinsonschen Krankheit.

## Claims

1. Compounds of formula (I): 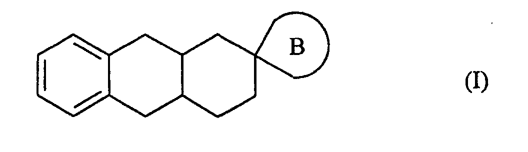 wherein
B represents an imidazoline ring as represented in formulae (Ia) and (Ib) : 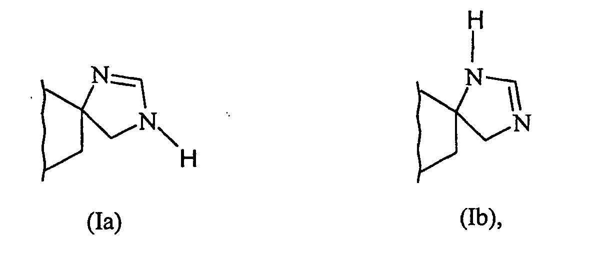 their tautomers, enantiomers and diastereoisomers, and addition salts thereof with a pharmaceutically acceptable acid or base.

2. Compounds of formula (I) according to claim 1 having a *trans* ring junction, their tautomers, enantiomers and diastereoisomers, and addition salts thereof with a pharmaceutically acceptable acid or base.

3. Compound of formula (I) according to claim 1 which is spiro[(1,3-diazacyclopent-1-ene)-5:2'-(*trans*-1',2',3',4',4'a,9',9'a,10'-octahydroahthracene)], its tautomers, enantiomers and diastereoisomers, and addition salts thereof with a pharmaceutically acceptable acid or base.

4. Compound of formula (I) according to claim 1 composed of the mixture of spiro-[(1,3-diazacyclopent-1-ene)-5:2'*(S)*-(*trans*-1',2',3',4',4'a*(R)*,9',9'a*(S)*,10'-octahydroanthracene)] and spiro[(1,3-diazacyclopent-1-ene)-5:2'*(R)*-(*trans*-1',2',3',4',4'a*(S)*, 9',9'a*(R)*,10'-octahydroanthracene)], its tautomers, enantiomers and diastereoisomers, and addition salts thereof with a pharmaceutically acceptable acid or base.

5. Compound of formula (I) according to claim 1 composed of the mixture of spiro-[(1,3-diazacyclopent-1-ene)-5:2'*(S)*-(*trans*-1',2',3',4',4'a*(S)*,9',9'a*(R)*,10'-octahydroanthracene)] and spiro[(1,3-diazacyclopent-1-ene)-5:2'*(R)*-(*trans*-1',2',3',4',4'a*(R)*, 9',9'a*(S)*,10'-octahydroanthracene)], its tautomers, enantiomers and diastereoisomers, and addition salts thereof with a pharmaceutically acceptable acid or base.

6. Process for the preparation of compounds of formula (I) according to claim 1 **characterised in that** there is used as starting material a compound of formula (II) : 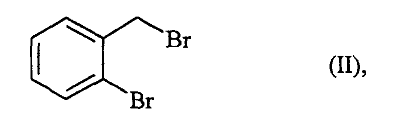 which is condensed with 1,4-cyclohexanedione mono-ethylene acetal enolate in order to obtain a compound of formula (III) : 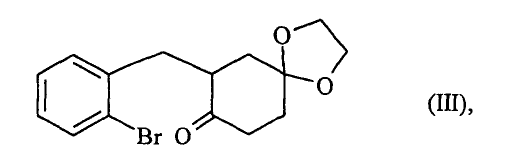 which is subjected to the action of methyl(triphenyl)phosphonium iodide in basic medium to yield a compound of formula (IV) : 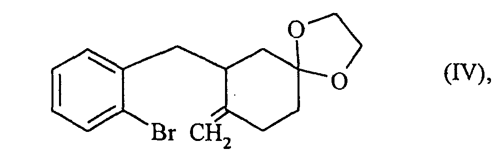 which is cyclised in the presence of tributyltin hydride and AIBN to yield a compound of formula (V) : 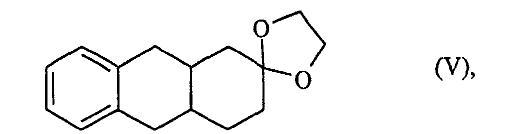 which is subjected, in succession, to the action of an acidic medium followed by a Strecker reaction to obtain a compound of formula (VI) : 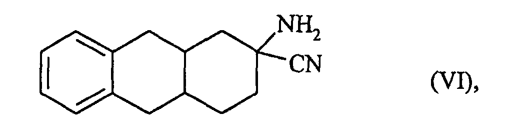 which is subjected to the action of a reducing agent to yield a compound of formula (VII) : 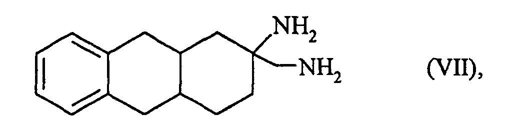 which is reacted with formamidine acetate to obtain a compound of formula (I/a), a particular case of the compounds of formula (I): 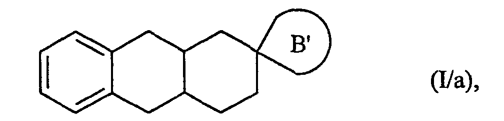 wherein B' represents an unsubstituted imidazoline ring as represented in the formulae (Ia/a) and (Ib/a) : 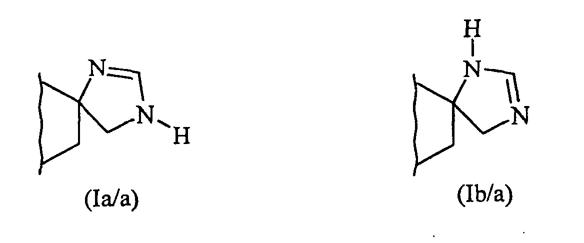 which may be subjected, in the presence of a base, to the action of a compound of formula (VIII) :
R'-J (VIII)
wherein R' represents a linear or branched (C₁-C₆)alkyl group or a benzyl group, and J represents a leaving group, such as a halogen atom or a tosyl group, to yield a compound of formula (I/b), a particular case of the compounds of formula (I) : 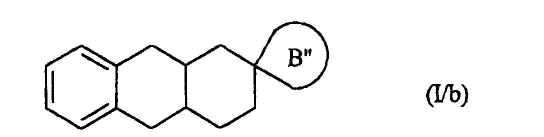 wherein B" represents a substituted imidazoline ring as represented in the formulae (Ia/b) and (Ib/b) : 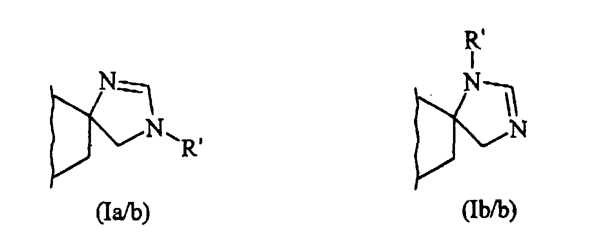 wherein R' is as defined hereinbefore,
which compounds of formulae (I/a) and (I/b) constitute the totality of the compounds of formula (I) and may be purified according to a conventional separation technique, are converted, if desired, into their addition salts with a pharmaceutically acceptable acid or base, and are separated, where appropriate, into their isomers according to a conventional separation technique.

7. Pharmaceutical compositions comprising as active ingredient at least one of the compounds of formula (I) according to any one of claims 1 to 5 or an addition salt thereof with a pharmaceutically acceptable acid or base in combination with one or more pharmaceutically acceptable excipients.

8. Pharmaceutical compositions according to claim 7 for use in the production of a medicament for the treatment of depression, obesity, panic attacks, anxiety, obsessive-compulsive disorders, cognitive disorders, phobias, impulsive disorders associated with the abuse of drugs and withdrawal therefrom, sexual dysfunctions and Parkinson's disease.
